# EUROPEAN PATENT APPLICATION

(11) **EP 3 660 856 A1**
(43) Date of publication of application: **03.06.2020**
(21) Application number: 18208771.8
(22) Date of filing: 28.11.2018
(51) Int. Cl.: G16H 20/17

(54) **AUGMENTED REALITY FOR DRUG DELIVERY DEVICES**

(71) Applicant: Tecpharma Licensing AG, 3401 Burgdorf (CH)
(72) Inventor: Kühni, Florian, 3007 Bern (CH); Reubi, Dominik, 3012 Bern (CH)

(57) **Abstract**

The invention concerns a computer implementable method for supporting an operation of a drug delivery device (1) comprising the steps of capturing, by a camera of a mobile device (4), a marker (3) representative of a position of the drug delivery device (1); transmitting, by the mobile device (4), camera data of the camera relating to the captured marker (3) to a provider (5); providing in response to the transmitted camera data, by the provider (5), drug delivery device data to the mobile device (4); displaying, by the mobile device (4), in a camera preview the provided drug delivery device data, wherein the displaying is controlled by a projection of the marker (3). The drug delivery device data comprises instructions to a user relating to an operation of the drug delivery device (1).

## Description

### FIELD OF THE INVENTION

The present invention relates to drug delivery systems for delivering, administering, injecting, infusing or dispensing liquids comprising a drug, medicament, or active ingredient. Preferred variants of the invention depart from a method of supporting an operation of a drug delivery device by displaying, by a mobile device, in a camera preview drug delivery device data.

### BACKGROUND

A variety of diseases exist that require regular treatment by subcutaneous administration of a medicament, and a number of drug delivery devices have been developed to support a patient in accurately and controllably delivering an amount of drug in a self-administration process.

Medical systems are known in the art that use augmented reality for guiding a physician through a surgical procedure. Such system comprise glasses with a display showing steps or information. For example, US 9,832,412 B2 discloses a system that allows the physician operating in a hands-free manner using glasses with a camera and an augmented reality display. The glasses are configured to identify and recognize a syringe, which includes an identification tag such as a QR code. The identification tag provides information about the syringe and the fluid contained therein such as medication type, total fluid volume, manufacturer, needle dimensions and fluid expiration date. Depending on its position the identification tag can be used to estimate the position of the plunger rod of the syringe. The detected information can be displayed on a virtual layer projected over the user field of view in the display of the glasses. Besides that, information including time and date of the injection can be recorded by the glasses and transmitted to an external patient data system. Furthermore, the virtual layer can assist the physician in positioning the syringe by providing a virtual trace of the location of a vein suitable for needle insertion. The virtual trace is a computer-generated image. The position of the vein can be determined by images captured by a camera of the glasses allowing to identify the anatomic position of portion of the arm of the patient.

However, such supporting by augmented reality system is reserved physicians or health care professionals in a hospital environment. Besides that the system requires sophisticated and expensive glasses and corresponding software programs.

Another application of an augmented reality system discloses WO 2018/065883 A1 with a system for training and monitoring administration of an inhaler medication. A patient application implemented on a user mobile phone is programmed to utilize the mobile phone camera and microphone to record video and audio data. The application displays the real-time video to the patient on the display and overlays or renders additional digital content on the screen so as to provide an augmented reality tutorial to the patient. The application is further configured to analyze the real-time video and audio data and evaluate the patient's technique for administering medication using the inhaler and dynamically update and modify the instruction accordingly. Furthermore, the patient application is in communication with a back-end system server via network.

These prior art approaches are limited to finding a puncture site by showing the vein, or by the technique for placing an inhaler on the face. The guidance is specific and presume a knowledge of the user for operating the delivery device or the inhaler.

### SUMMARY OF THE INVENTION

It is an objective of the invention to enable an easy and cost-effective entirely supporting of a user for operating a drug delivery device. This objective is achieved by a method, a computer program product and a computing device according to the independent claims. Preferred embodiments are evident from the dependent patent claims.

According to the invention a computer implementable method for supporting an operation of a drug delivery device, in particular an injection device or an infusion device, comprises the steps of
a. Capturing, by a camera of a mobile device, a marker representative of a position of the drug delivery device;
b. Automatically transmitting, by the mobile device, camera data of the camera relating to the captured marker to a provider;
c. Providing in response to the transmitted camera data, by the provider, drug delivery device data to the mobile device;
d. Displaying, by the mobile device, in a camera preview the provided drug delivery device data, wherein the displaying is controlled by a projection of the marker;

The drug delivery device data comprises instructions to a user relating to an operation of the drug delivery device, in particular a drug delivery.

The camera recognizes the marker, which is representative of the position and orientation of the drug delivery device relative to the mobile device. The marker may be an image, sign or symbol on the housing or shell of the drug delivery device. For example, the marker may be an image with a characterizing black frame or any other anchoring point, specific sign, logo, letter or the like. In another embodiment the outer contour of the drug delivery device itself may form the marker recognizable by the camera. In any case, the marker can be recognized quick and reliable by the camera of the mobile device.

The marker is representative of the position and orientation of the drug delivery device. That means based on the data captured by the camera a software program is able to determine the position and/or orientation of the drug delivery device relative to the mobile device and relative to other objects captured by the camera. Thus, with the marker the software may arrange augmented reality content in a display relative to the drug delivery device.

After capturing the marker, the mobile device transmits by wire or wireless the captured camera data relating to the marker to a provider. Preferably, the mobile device automatically transmits the data to the provider without any manual user input.

The provider is independent of the mobile device and is preferably a drug delivery support service provider, in particular a web application or a web appliance hosted by a web server. The provider may be implemented as software only or the provider may comprise software and also hardware. However, the provider is not limited to web application or web appliance. In contrast, the provider may be any software and/or hardware adapted to provide information on request to the mobile device. For example, the provider may be a service provider including e. g. human personal, wherein the service provider is connected over a local, a wide area network or over the internet with the mobile device.

The provider provides in response to the transmitted camera data and based on the captured marker drug delivery device data to the mobile device. Preferably, specific data are assigned to a specific marker. Hence, the provider is able to provide marker-specific data to various markers. The drug delivery device data comprise instructions to the user relating to an operation of the delivery device.

The mobile device is preferably a portable, but non-wearable mobile device, for example, a mobile phone, a tablet computer, a laptop computer or the like. Alternatively, a wearable mobile device may be eyeglasses, which is in the present text not encompassed by the term "non-wearable" mobile device.

With the method according to the invention the user is guided or supported by augmented reality objects (AR objects) through the operation process of the drug delivery device in that instruction relating to the operation of the delivery device are displayed in the camera preview on the mobile device of the user. That means if the user points the camera of his mobile device to the drug delivery device the user sees the drug delivery device together with AR objects in the form of instructions for operating the device in the camera preview of his mobile device. The AR objects, namely the drug delivery device data, are preferably made visible to the user on a display layer displayed in the camera preview. Therefore, the captured real time camera view and also the overlaid instructions are shown in the same display at the same time. The AR objects are preferably dynamically arranged. That means, if the user moves the camera around the drug delivery device the AR objects in the display are rearranged based on a projection of the marker, such that the AR objects are placed at every time in an optimal position within the display of the camera preview with respect to the drug delivery device. Thus, the displaying of the data from the provider is controlled by a projection of the marker.

The instructions may be in the form of text, pictures, signs, pictograms or fully or partially transparent photographs or videos describing an operation of the drug delivery device. The instructions relating to the operation of the drug delivery device may be assigned to a specific delivery device. Thus, the provider provides only information that are relevant for the specific case based on the specific marker on the device. Additionally or instead, if the user has been authenticated the provider may provide user-specific instructions, for example, in a specific language or in a particular form, e. g. for a user with reduced visual capacity or limited motor skills.

The instruction relating to an operation of the drug delivery devices comprise, for example, instructions how to remove a needle shield or how to carrying out a priming, how to setting a dose or a trouble shooting guide. Furthermore, the augmented reality elements may be combined. For example, a text describing the setting of a dose may be combined with a sign or an arrow showing the user where on the drug delivery device the dose has to be set.

With the method according to the invention the user is guided or supported by augmented reality objects through operating process of the drug delivery device in that instruction are displayed in the camera preview on the mobile device of the user. Thus, the user can read and immediately carry out operating steps on the device. There is no need for reading a manual, consulting any other conventional operation documentation or requesting instructions from a human supervisor or instructor. That facilitates the drug delivery process and saves time. Furthermore, the user is able to focus on the drug delivery itself e.g. finding an appropriate puncture site. Besides that, the provided instructions in the camera preview may be used for training or education purposes. The user may practice the operation of the drug delivery device, for example, with a specific training device.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The term "drug delivery device" refers to a device for delivering, injecting, administering, infusing or dispensing substances and/or liquids from a product container into the skin of a patient. The injection may be, for example, intracutaneous, subcutaneous or intramuscular. The drug delivery device may be a disposable or a reusable drug delivery device. A disposable drug delivery device is a system, which is used for injecting the substance from a non-refillable and non-exchangeable cartridge. Once the amount of the substance to be injected either in one single or in several successive delivery events has been delivered the disposable drug delivery device is replaced by a new one. The cartridge in the disposable drug delivery device cannot be replaced. In comparison, the cartridge of a reusable drug delivery device may be replaced an arbitrary number of times.

The term "distal" refers to a direction where an injection needle is located and the term "proximal" is designating the opposite direction of the drug delivery device.

In the present description some of the steps of the method according to the invention are descripted as executed by the mobile device. However, it is to be understood that the mobile device does not carry out the steps on its own motion but rather the executed computer program causes the mobile device to carry out the mentioned steps. As mentioned above, the computer program is preferably residing and running on suitable storage and processing means of the mobile device.

In a preferred embodiment the instructions comprise operation information about setting a dose with the drug delivery device. Thus, the user is guided through the dose setting procedure by the instruction from the provider. This facilitates the drug delivery for the user, in particular if the user is not familiar with the operation of the drug delivery device.

Preferably, the provider provides device-specific instructions based on the camera data or the recognized marker, respectively. The instructions preferably comprise a user guide how to set the dose on the drug delivery device. Such a user guide may be in the form of a step by step guide. Namely, the instructions may comprise, for example, a sign or arrow showing the user the direction a possible dose button or dose sleeve of the drug delivery device has to be rotated in order to set a dose.

In another embodiment the instructions, for example, may indicate how to enter the desired dose in a user interface of the drug delivery device.

Instead or additionally, the instructions preferably comprise at least operation information about how to adjust settings of the drug delivery device, how to prepare the drug delivery device for an injection or an infusion, how to exchanging a cartridge in the drug delivery device, how to mount a needle, how to correcting a set dose, how to administer a set dose or disposal of the drug delivery device. As descripted the operation information are preferably in the form of a user guide.

The operation information, for example, may comprise instructions how to carry out a priming with the drug delivery device. For these instructions the data may comprise text, signs, pictures, photographs, videos or a combination thereof.

Additionally to the above mentioned operation information the instructions may comprise information about the injection process or the infusion process itself such as, for example, instructions how to place the drug delivery device onto the skin, how to insert a needle of the drug delivery device into the skin or where to place the drug delivery device on the body of the user. Furthermore, the instructions may comprise a counter showing the user the remaining time the drug delivery device to be hold on the injection site. Such injection or infusion information further facilitate the injection or infusion process for the user.

Preferably, the displaying of the drug delivery device data in the camera preview of the mobile device is implemented through a website displayed by a web browser of the mobile device. That means the displaying of the camera preview with the camera pictures is integrated in the website. Since the mobile devices usually comprises a web browser by default there is no need for an extra installation of an application on the mobile device. In other words, the mobile device is usually supplied with a preinstalled web browser ex works. Thus, there is no requirement for an installation and there are no additional expenses for the user in order to run the camera preview with the drug delivery device data from the provider.

In a preferred embodiment the drug delivery device is an injector, in particular a pen shaped injector or a patch injector. The method, the computer program and the mobile device according to the invention may be used with a disposable as well as with a reusable injector. Disposable injectors may be, for example, auto-injectors, autopen injectors or conventional fix dose or variable dose pen injectors. A patch injector is an injector which is adapted to be affixed onto the skin of the user and remains onto the injection site for the injection time, e.g. for several minutes.

The method according to the invention preferably comprises authentication process before the above descripted steps a - d. The authentication process preferably comprises the following steps:
I. Recognizing a machine-readable identification code and reading information from the code, by a sensor of the mobile device, wherein the code is assigned to the drug delivery device;
II. Automatically contacting, by the mobile device, the provider based on the read out information;
III. Sending, by the provider, in response to the contacting, an access request to data, in particular camera data, of the mobile device;
IV. Manually confirming, by the user via mobile device, the access request from the provider.

After the confirmation by the user the provider has access to the data and the provider can use the data for a further processing.

The machine-readable identification code may be realized in the form of a particular sign, symbol, letter or image such as a QR-code or the like. The sensor may be the camera of the mobile device or any other optical sensor adapted to read an identification code. The identification code is attached to or integrally formed with the drug delivery device. In an alternative embodiment the code is attached to or formed with the container or packaging of the drug delivery device.

Preferably, the mobile device automatically contacts the provider after the information has been read out from the identification code. That means no user input is required for contacting the provider. In a preferred embodiment the read out information of the code preferably prompts the mobile device to call up a website hosted by the provider. Subsequently, the provider requests access, preferably via a website, to the camera data of the mobile device. If the user confirms the request the provider uses the camera data for building up the camera preview, preferably integrated in the website. The camera preview is then displayed on the mobile device, in particular via browser, to the user. The authentication process ensures that the user can control the access of the camera data of his mobile device.

Independent whether or not the above descripted authentication process is implemented the method according to the invention preferably comprises a method for transmitting read out information form the identification code to the provider. If an authentication as descripted above is implemented the transmitting of the read out information is preferably subsequent to the authentication. In a preferred embodiment the method comprises the following steps
i. Recognizing the machine-readable identification code and reading information from the code, by a sensor of the mobile device, wherein the code is assigned to the drug delivery device;
ii. Transmitting, by the mobile device, read out information from the identification code to the provider;
iii. Providing, by the provider, in response to the received information at least one of data relating to the drug delivery device and data relating to the medication in the drug delivery device;
iv. Displaying, by the mobile device in the camera preview as descripted above, the data provided by the provider.

Preferably, the read out information prompt the mobile device automatically without manual user input to transmit the read out information to the provider. The data relating to the drug delivery device provided by the provider may comprise, for example, the type of the delivery device, the size of the delivery device, the lot number or the expiration date of the drug delivery device, if any. Such device information may help the user to verify whether he or she has chosen the correct drug delivery device.

Preferably, the data relating to the medication comprise at least one of the following information; medication type, expiration of medication, or validity of medication. The validity of medication may be verified through the provider if the provider has access to a database or to data from the drug manufacturer. In this case, the provider may provide a warning to the user if the medication is recalled. The warning can be displayed in the camera preview with the other provider data. Therefore, the provider may prevent the user from administering a recalled medication.

In a preferred embodiment the method additionally comprises the following steps:
v. Comparing, by the provider, the information about the medication with previously stored user-specific medication data;
vi. Providing, by the provider, a result of the comparison to the mobile device;
vii. Displaying, by the mobile device in the camera preview, the result of the comparison.

In order to be able to compare user-specific data the user has to be identified by the provider. For that purpose, the user may be prompted to log in via website or the mobile device may transmit a user identification to the provider along with the camera data.

The user-specific medication data, for example, may comprise a therapy plan or an indication of the dose to be administered to the specific user and/or the type of the medication to be used for the user. Such user-specific data may be stored in a database accessible by the provider or in storage means of the provider.

Preferably, after a user identification the provider only sends information relevant for the identified user to the mobile device.

The provider may verify if the user is about to use the correct medication based on the identification code and/or based on the camera data. For that purpose, the provider may compare, for example, captured camera data of the identification code, the medication or the marker with the previously stored user-specific data. If the comparison reveals that the user is about to take the medication according to the stored user-specific data the provider may, for example, send a confirmation, e. g. a green approved sign, which is displayed in the camera preview. In case the comparison reveals that the captured information about the medication does not correspond to the stored user data the provider may send a warning or alert to the user via camera preview in the mobile device.

Additionally, in this case the provider may send a warning to any other external receiver, for example, to a health care practitioner. The comparison by the provider increases the safety and reduces the risk an inappropriate medication is administered to the user.

Additionally, to the above mentioned comparison the provider may provide, for example, a reminder or an alert to the user to administer a drug based on a user-specific timetable. Such alerts from the provider are preferably displayed in the camera preview and dynamically controlled by the projection of the marker.

In a preferred embodiment the drug delivery device comprises an electronic module which is integrated in or attachable to the drug delivery device and is adapted for monitoring of a drug delivery process executed by means of the drug delivery device. Preferably, the method comprises the steps of
I. Recording, by the electronic module, delivery data related to the drug delivery process;
II. Transmitting the delivery data from the electronic module to the mobile device;
III. Displaying, by the mobile device, in a camera preview the delivery data, wherein the displaying is dynamically controlled by a projection of the marker.

The data are preferably transmitted from the electronic module to the mobile device and from there to the provider. The mobile device, preferably via browser, displays the delivery data in the camera preview. The displaying is controlled by a projection of the marker, e.g. arranged in relation to the drug delivery device. Preferably, the delivery data are transmitted via a short range communication such as, for example, Bluetooth, Bluetooth Low Energy or LTE Cat M1, from the electronic module to the mobile device. The mobile device may regularly request data from the electronic module and forwards the delivery data to the provider.

In an alternative embodiment the provider may collect delivery data from a database or any a storage medium, for example, form a cloud storage. The database with the user-specific delivery data may be build up by the user, for example, if the user keeps a diary or a personal therapy plan or the data may be entered in the database by a health care professional.

In case the provider collects the delivery date from an external database the user has to be identified. As mentioned above the user may be prompted to log in to the provider. In an alternative embodiment the mobile device may, after approval by the user, transmit a user identification along with the camera data to the provider.

Preferably, the delivery data related to the drug delivery process comprise at least one of the following information; an amount of the last administered dose, indication of time of the last administered dose, amount of the next dose to be administered, indication of time of the next dose to be administered or total amount of administered dose.

In case the provider receives the delivery data directly from an electric module of the drug delivery device having a temperature sensor the delivery data may further comprise a temperature of medication.

In a preferred embodiment the drug delivery device may be configured by AR operation elements or by AR control elements. In this case, the mobile device is preferably connected via communication link to the electronic module integrated in or attachable to the drug delivery device. Preferably, the mobile device receives the instruction from the user via AR control elements and sends configuration data to the electronic module.

The AR operation elements or AR control elements may be, for example, realized in the form of a gesture control. That means gestures from a hand of the user are captured by the camera of the mobile device. The camera data are sent to the provider or processed in the mobile device. The provider or an AR software on the mobile device recognizes the gestures and sends configuration data related to a specific gesture to the electronic module. Alternatively, the AR control elements may be realized in the form of a voice control. In this case, the user speaks a command which is captured by a microphone of the mobile device. After recognizing the user command, the mobile device sends the configuration data related to the specific command to the electronic module.

The configuration data may comprise, for example, commands to activate or enable the electronic module (e. g. disabling a sleep mode of the electronic module), setting the drug delivery device in a delivery mode, such that the drug delivery device is ready for use, automatically priming the drug delivery device, automatically set a dose in the drug delivery device or showing delivery data such as, for example, the time and amount of the last administered dose or the medication type being inside the drug delivery device on a display of the electronic module.

Preferably, the drug delivery device can be configured by the user only after a successful authentication process based on the recognized identification code as descripted above.

In a further preferred embodiment the method comprises the steps of pairing the mobile device and a remote computing device of a human expert and transmitting, by the mobile device, camera data captured by the camera of the mobile device to the remote computing device.

The remote computing device of the human expert may be, for example, a desktop computer, a mobile phone, a laptop computer, a tablet computer or a server system. The human expert may be a doctor, a health care practitioner or a device engineer which is usually not in the immediate proximity to the user.

Preferably, the user has to confirm that the mobile device sends captured camera data to the remote device of the expert. Due to the camera data the external expert is able to analyze or verify the situation of the user. For example, the expert can verify if the user uses the drug delivery device in a correct manner or if the user is about to administer the correct medication with a dose at a time according to a predefined user-specific therapy plan or predefined process. That means the user is not left alone and the expert can influence the administration process.

Furthermore, in a preferred embodiment the method comprises the steps of transmitting, by the remote computing device, instructions from the human expert to the mobile device of the user and displaying, by the mobile device in the camera preview, the instructions of the expert.

Thus, the user can see the instructions in the camera preview. Preferably, the instructions from the expert are displayed in real time. That means, if the expert recognizes an action of the user or a condition that need to be commented or guided the expert may provide information or advise via the remote computing device. Since such data from the expert are displayed in the camera preview the user can focus on the camera preview and does not have to switch to any other device or display. This is advantageous in particular for a user suffering from reduced physical, sensory or mental capabilities.

The information or instructions from the expert may be displayed in the camera preview of the mobile device of the user together with the above descripted delivery date and/or medication data provided by the provider. Thus, the user can see all necessary information or instructions in the camera preview. This facilitates the operation of the drug delivery device and thus the whole injection of infusion process.

In an further preferred embodiment, the method comprises the step of outputting, by the mobile device, an acoustic signal based on the drug delivery device data received from the provider. Preferably, the drug delivery data from the provider are not only displayed in the camera preview but additionally the information or instructions are acoustically outputted to the user via loudspeaker. The acoustic signal may be sent by the provider in case of a warning or an alarm. Hence, the alarm is not only displayed in the camera preview but additionally outputted as an acoustic signal to the user. This allows to output an alarm which causes more attention.

Furthermore, the acoustic signal may comprise instructions to the user in spoken form. Thus, the user receives the instructions via voice output of the mobile device such that the user can listen to the spoken instructions.

The invention relates further to a computer program product, in particular a web program, comprising instructions which, when the program is executed by a computing device, cause the computing device to carry out the steps of:
a. Receiving, from a mobile device, camera data of a camera of the mobile device relating to a captured marker, wherein the marker is representative of a position of the drug delivery device;
b. Providing, in response to the received camera data, drug delivery device data to the mobile device; wherein the drug delivery device data comprises instructions to a user relating to an operation of the drug delivery device, in particular a drug delivery operation of the drug delivery device.

The invention relates further to a computer program product comprising instructions which, when executed by a computing device, cause the computing device to carry out the steps of:
a. Capturing, by a camera of a mobile device, a marker representative of a position of the drug delivery device;
b. Transmitting, by the mobile device, camera data of the camera relating to the captured marker to a provider;
c. Receiving in response to the transmitted camera data, drug delivery device data from the provider;
d. Displaying, by the mobile device, in a camera preview the provided drug delivery device data, wherein the displaying is controlled by a projection of the marker;
wherein the drug delivery device data comprises instructions to a user relating to an operation of the drug delivery device, in particular a drug delivery operation of the drug delivery device.

Furthermore, the invention relates to a mobile device comprising processing means configured for carrying out the steps of the computer program product as descripted above. The mobile device is preferably a mobile phone. Alternatively, the mobile device may be tablet computer, a laptop computer, a handheld computer, a PDA or the like.

The invention is further related to the use of the computing device comprising processing means configured for carrying out the steps
a. Capturing, by a camera of a mobile device, a marker representative of a position of the drug delivery device;
b. Transmitting, by the mobile device, camera data of the camera relating to the captured marker to a provider;
c. Receiving in response to the transmitted camera data, drug delivery device data from the provider;
d. Displaying, by the mobile device, in a camera preview the provided drug delivery device data, wherein the displaying is controlled by a projection of the marker; Displaying, by the mobile device, in a camera preview the provided drug delivery device data, wherein the displaying is controlled by a projection of the marker; wherein the drug delivery data comprises instructions to a user relating to an operation of the drug delivery device, in particular a drug delivery operation of the drug delivery device.

### BRIEF DESCRIPTION OF THE DRAWINGS

The subject-matter of the invention will be explained in more detail in the following text with reference to preferred exemplary embodiments as illustrated in the attached drawings, of which
- Fig. 1: schematically depicts an arrangement with a injector, a web server, user mobile phone connected to the internet and displaying AR objects from an AR web application;
- Fig. 2: schematically depicts the arrangement of Figure 1 additionally with a connected computing device of an external expert.

### DETAILED DESCRIPTION OF PREFERRED EMBODIMENTS

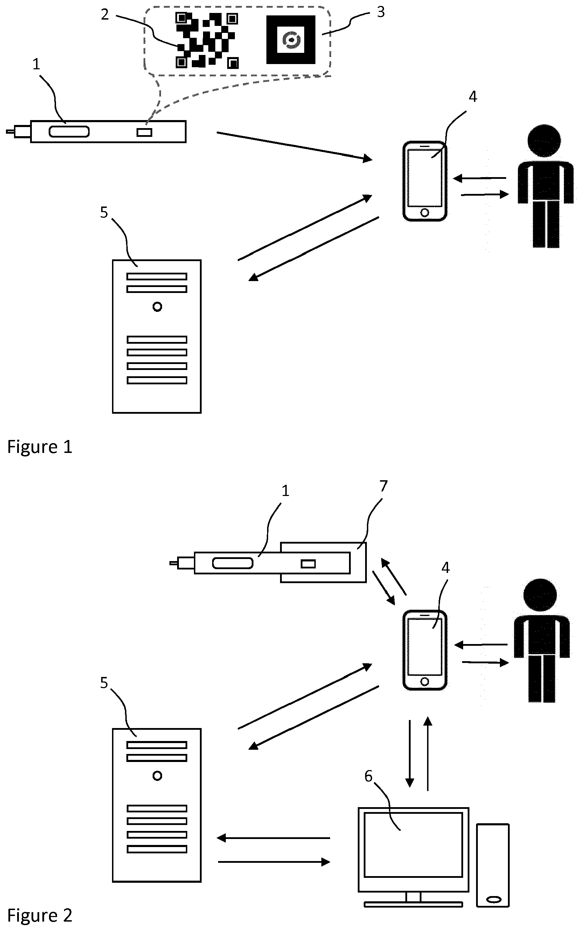

In the following description a guidance of an injection process by augmented reality objects according to the invention is descripted. Figure 1 schematically depicts a drug delivery device in form of a pen injector 1, a web application 5 running on a web server and a mobile device in the form of a user mobile phone 4 comprising a camera and a display. A camera preview is displayed in on the display of the mobile phone 4. The flow of data is shown with arrows.

In order to start an augmented reality guided operation of the injector 1, the user activates the camera of the mobile phone 4 or opens a QR code reader App. The user looks up in the camera preview a machine-readable identification code such as a QR-code 2, which is attached to the housing of the injector 1. The camera of the mobile phone 4 is used as an optical sensor to recognize the QR-code 2. Alternatively, a specific QR code application running on the mobile phone 4 together with the camera can be used to recognize the QR code 2 on the injector 1.

If the QR code is recognized and read by the camera the mobile phone 4 is prompted to call via internet connection an augmented reality (AR) web application 5 (web program) hosted by a web server. The mobile phone 4 automatically calls the AR web application 5 and displays an AR website via standard web browser to the user. The AR web application 5 requests access to camera data captured by the camera of the mobile device 4. After the user confirms that access the browser is able to collect the camera data and can display the camera data on the mobile phone 4 to the user.

In a preferred embodiment a standard mobile web browser such as Chrome, Opera or Firefox may be used for displaying the AR website with the AR objects. That means there is no need for an extra or specific installation of an AR application on the mobile phone 4. The standard web browser is preinstalled on the mobile phone 4 ex works. Alternatively, a specific AR mobile application can be installed on the mobile phone 4 to display the AR content to the user.

After the authentication process the camera preview is integrated in the displayed AR website. Hence, the browser displays the camera preview to the user. Then, the user captures a marker 3 or pattern, for example, in the form of a black-framed sign on the housing of the injector 1 with the camera of the mobile phone 4. After recognizing the marker 3, the AR web application 5 receives the camera data and it sends marker-specific AR objects to the mobile phone 4.

Along with the AR objects the AR web application 5 sends commands in form of a script to the browser on the mobile phone 4. Based on the script the browser places the AR objects in real time on a visual layer relative to the captured marker 3 in the camera preview. That means the user sees the AR objects in the camera preview on a AR layer. The AR objects are placed next to, above, beneath or overlapping the injector 1 in the camera preview. Additionally, the AR content may comprise acoustic signals, such as spoken instructions which are outputted via loudspeaker of the mobile phone 4 to the user.

As long as the user holds the camera of the mobile phone 4 in direction to the injector 1 such that the marker 3 can be recognized by the camera the instructions are dynamically displayed on the AR layer in the camera preview next to the injector 1. When the user moves the mobile phone 4 but still focus on the marker 3 the AR objects are moved too in the camera preview such that the AR objects keep a defined optical relationship to the marker 3. That means, for example, if the camera is moved to the right such that the injector 1 is displayed at the left edge the AR object moves too to the left edge in the camera preview. Furthermore, if the AR objects are three-dimensional objects and if the injector 1 is captured in a other spatial orientation the spatial orientation of the AR objects changes accordingly.

In the following a use case with the method according to the invention is descripted in detail.

After the above descripted authentication process with the user and the capturing of the marker on the injector the AR web application prompts the browser on the mobile phone to place and display in the camera preview the AR objects in form of instruction to the user relating to an operation of the injector. Based on the camera data the AR web application recognizes which marker the user has captured. Accordingly, the application provides marker-specific instructions. For example, the user captures with the camera a marker on the housing of an autoinjector. In this case, the AR web application provides operating instructions for operating the autoinjector.

In order to guide the user through the operating steps the AR web application displays via browser in the camera preview a short text how to decap the autoinjector together with an arrow pointing to the cap of the autoinjector. After a certain time or after the user has confirmed with a manual input on the mobile phone that the decapping step has been carried out the AR web application provides the next instruction. The next steps are how to hold the autoinjector onto the skin and how to start the injection with the autoinjector. Again, the provided instructions comprise a text and an arrow symbol indicating the user the direction to push the autoinjector in order to start the injection. If the user confirms the starting of the injection via browser or mobile phone the AR web application provides a counter, which shows the remaining time the autoinjector has to be hold on the injection site and which is displayed as an AR object in the camera preview. When the counter has elapsed the AR web application may send a short description shown in the camera preview how to dispose the autoinjector.

In another embodiment the method according to the invention may be used for a conventional disposable injection pen, for example, for a disposable insulin pen.

To start the injection the AR web application provides instruction how to carry out the priming with the pen. For this purpose, the application prompts the browser on the mobile phone to show a short video sequence displayed next to the pen in the camera preview. The video sequence shows the user where and in what direction the dose button of the pen has to be rotated and how to push the release button. After a user confirmation via browser the AR web application provides instructions how to set a dose and how to correct a dose with the pen. The instructions comprise a textbox, arrows and signs dynamically placed in relation to the captured marker in the camera preview. Subsequently, the instructions how to place the pen onto the skin and how to push the release button in order to start the injection may be shown in the camera preview.

Additionally to the above mentioned instructions the user may be guided by AR objects for one or more of the following operation steps: adjusting settings of the injector, preparing the injector for an injection, exchanging a cartridge in the injector or mounting a needle to the injector.

In a further embodiment the method according the invention may also be used for a medical infusion pump, for example, for a conventional insulin pump, a patch pump or a semi disposable pump. The instructions provided by the AR web application may comprise in this case information to the user how to set a dose on a user interface on the pump, e. g. a touch screen, how to change pump parameters on the pump, how to exchange a cartridge in the pump, how to carry out a priming with the pump, how to prepare a patch pump for an attachment to the body of the user or how to exchange the disposable part of a semi disposable pump.

Furthermore, in another embodiment the instructions are related to a patch injector, which is adhesive attachable onto the skin of the user and remains thereon for several minutes until the injection is completed.

Besides instructions relating to the operation of the injector the AR web application may additionally provide information about the injector and the medication in the camera preview based on a captured identification code. Furthermore, the AR web application may further provide users-specific information based on stored user data, in particular based on a user-specific therapy plan.

That means the user itself or a health care practitioner creates a user profile and enters the user-specific information and therapy plan via a web portal. The user data are stored in a database on a web server, for example, in a cloud storage. The user-specific therapy plan includes information about the medication to be administered and date and time of the planned administration.

The user has to log in to the AR web application and to authorize the AR web application such that the application has access to the user data including the therapy plan. Alternatively, the user-specific data in form of a user ID or the like are transmitted from the mobile phone to the AR web application in order to authorize the AR web application for the user data.

The user reads by the camera of the mobile phone a machine-readable identification code in form of a QR-code on the housing of the injector, on the cartridge or on the packaging of the injector. The mobile phone transmits the read out information to the AR website which extracts the information and displays the extracted information about the injector and the medication in the camera preview as AR objects.

The information about the injector comprise an indication of the type of the injector and if available an expiration date of the injector. The information about the medication comprise medication type and expiration of medication.

The AR web application then compares the data based on the read out information from the QR-code with the data stored in the user profile. If the chosen injector and/or the chosen medication corresponds to the previously defined therapy data the AR web application displays a confirmation (for example a green tick) via browser in the camera preview on the mobile phone. In case the injector and/or medication does not correspond to the therapy plan or to predefined conditions a warning or alert (for example a red exclamation mark) is displayed in the camera preview. Furthermore, the AR web application verifies if the medication had not yet expired and if the medication had not been recalled. If so, a warning is displayed in the camera preview.

Figure 2 schematically depicts a further embodiment according to the invention. In this embodiment the method comprises additionally the step of collecting data from an electronic module in the form of an add-on 7 releasably attachable to an injector 1. The add-on 7 is adapted for monitoring of an injection process executed by the injector 1. For that purpose, the add-on 7 records delivery data such as the injected amount of the medication and the date and time of the injection.

The AR web application or the mobile phone periodically request the delivery date from the add-on. The delivery data are transmitted via Bluetooth Low Energy connection from the add-on to the mobile phone. Subsequently, the browser displays the collected delivery data on the mobile phone to the user. The mobile phone further transmits the delivery data to the user profile on an external server and stores the data. In order to support or remind the user the AR web application displays via browser additional data from the user profile or from the user therapy plan such as the amount of the next dose to be administered or the time of the next dose to be administered.

As depicted in the figure 2 the mobile phone 4 of the user can be additionally connected to a remote computer 6 of an external expert, for example, a health care practitioner (HCP).

In this case, the user starts a paring of his mobile phone with the remote computer such as a desktop computer, a laptop, a mobile phone, a tablet computer or the like of the HCP. After a pairing and authentication process the HCP can access via AR web application to the camera data captured by the camera of the mobile phone of the user. Thus, the HCP can see the camera preview including the AR layer as displayed on the mobile phone of the user. As the HCP can see the user actions through the camera the HCP can make an impact to the operating process in real time by providing information or guidance, either via text messages or through a telephone connection. For that purpose, the AR web application may provide a chat room and the user may give access to the HCP to the chat room in order to be able to communicate with the HCP.

### LIST OF REFERENCE NUMERALS

- 1: Injector
- 2: QR code
- 3: Marker
- 4: Mobile phone
- 5: Web application
- 6: Remote computer
- 7: Add-on

## Claims

1. A computer implementable method for supporting an operation of a drug delivery device (1), in particular an injection device or an infusion device, comprising the steps of
a. Capturing, by a camera of a mobile device (4), a marker (3) representative of a position of the drug delivery device (1);
b. Transmitting, by the mobile device (4), camera data of the camera relating to the captured marker (3) to a provider (5);
c. Providing in response to the transmitted camera data, by the provider (5), drug delivery device data to the mobile device (4);
d. Displaying, by the mobile device (4), in a camera preview the provided drug delivery device data, wherein the displaying is controlled by a projection of the marker (3);
**characterized in that**
the drug delivery device data comprises instructions to a user relating to an operation of the drug delivery device (1), in particular a drug delivery operation of the drug delivery device (1).

2. Method according to claim 1, wherein the instructions comprise operation information about setting a dose with the drug delivery device (1).

3. Method according to claim 1 or 2, wherein the instructions comprise operation information about adjusting settings of the drug delivery device (1), preparing the drug delivery device (1) for an injection or an infusion, exchanging a cartridge in the drug delivery device (1), mounting a needle, correcting a set dose, administering a set dose or disposal of the drug delivery device (1).

4. Method according to any of claim 1 to 3, wherein the displaying of the drug delivery device data in the camera preview is implemented through a website displayed by a web browser of the mobile device (4).

5. Method according to any of claim 1 to 4, wherein the drug delivery device (1) is an injector, in particular a pen shaped injector or a patch injector.

6. Method according to any of claim 1 to 5 comprising, before step a) of claim 1, an authentication process including the steps of
I. Recognizing a machine-readable identification code (2) and reading information from the code (2), by a sensor of the mobile device (4), wherein the code (2) is assigned to the drug delivery device (1);
II. Automatically contacting, by the mobile device (4), the provider (5) based on the read out information;
III. Sending, by the provider (5), in response to the contacting, an access request to data, in particular camera data, of the mobile device (4);
IV. Manually confirming, by the user on the mobile device (4), the access request from the provider (5).

7. Method according to any of claim 1 to 6 comprising the steps of
i. Recognizing a machine-readable identification code (2) and reading information from the code (2), by a sensor of the mobile device (4), wherein the code (2) is assigned to the drug delivery device (1);
ii. Transmitting, by the mobile device (4), read out information from the identification code (2) to the provider (5);
iii. Providing, by the provider (5), in response to the received information at least one of data relating to the drug delivery device (1) and data relating to the medication in the drug delivery device (1);
iv. Displaying, by the mobile device (4) in the camera preview of claim 1d), the data provided by the provider (5).

8. Method according to claim 7, wherein the data relating to the medication comprise at least one of the following information; medication type, expiration of medication, or validity of medication.

9. Method according to claim 7 or 8, comprising the steps of
v. Comparing, by the provider (5), the information about the medication with previously stored user-specific medication data;
vi. Providing, by the provider (5), a result of the comparison to the mobile device (4);
vii. Displaying, by the mobile device (4) in the camera preview of claim 1d), the result of the comparison.

10. Method according to any of claim 1 to 9, wherein an electronic module (7) integrated in or attachable to the drug delivery device (1) is adapted for monitoring of a drug delivery process executed by means of the drug delivery device (1), wherein the method comprises the steps of
I. Recording, by the electronic module (7), delivery data related to the drug delivery process;
II. Transmitting the delivery data from the electronic module (7) to the mobile device (4);
III. Displaying the delivery data, by the mobile device (4), in a camera preview, wherein the displaying is dynamically controlled by a projection of the marker (3).

11. Method according to claim 10, wherein the delivery data related to the drug delivery process comprise at least one of the following information; an amount of the last administered dose, indication of time of the last administered dose, amount of the next dose to be administered, indication of time of the next dose to be administered or total amount of administered dose.

12. Method according to any of claim 1 to 11 comprising the steps of pairing the mobile device (4) and a remote computing device of an expert and transmitting, by the mobile device, camera data captured by the camera of the mobile device (4) to the remote computing device (6).

13. Method according to any of claim 1 to 12 comprising the step of outputting, by the mobile device (4), an acoustic signal based on the drug delivery device data received from the provider (5).

14. Computer program product, in particular a web program, comprising instructions which, when the program is executed by a computing device, cause the computing device to carry out the steps of:
a. Receiving, from a mobile device (4), camera data of a camera of the mobile device (4) relating to a captured marker (3), wherein the marker (3) is representative of a position of the drug delivery device (1);
b. Providing, in response to the received camera data, drug delivery device data to the mobile device (4);
**characterized in that**
the drug delivery device data comprises instructions to a user relating to an operation of the drug delivery device (1), in particular a drug delivery operation of the drug delivery device (1).

15. The use of a mobile device (4) comprising processing means configured for carrying out the steps
a. Capturing, by a camera of the mobile device (4), a marker (3) representative of a position of a drug delivery device (1);
b. Transmitting, by the mobile device (4), camera data of the camera relating to the captured marker (3) to a provider (5);
c. Receiving in response to the transmitted camera data, drug delivery device data from the provider (5);
d. Displaying, by the mobile device (4), in a camera preview the provided drug delivery device data, wherein the displaying is controlled by a projection of the marker (3); wherein the drug delivery device data comprises instructions to a user relating to an operation of the drug delivery device (1), in particular a drug delivery operation of the drug delivery device (1).
